# EUROPEAN PATENT APPLICATION

(11) **EP 4 801 235 A1**
(43) Date of publication of application: **02.09.2026**
(21) Application number: 24882490.6
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT, PHOTOELECTRIC CONVERSION DEVICE, MOBILE BODY, AND BUILDING MATERIAL**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086010
(71) Applicant: Canon Kabushiki Kaisha, Tokyo 146-8501 (JP)
(72) Inventor: MAKISUMI, Kohei, Tokyo 146-8501 (JP); SEKIDO, Kunihiko, Tokyo 146-8501 (JP); KATO, Nanami, Tokyo 146-8501 (JP); YOSHIDA, Yu, Tokyo 146-8501 (JP); NISHIDA, Tsutomu, Tokyo 146-8501 (JP)
(74) Representative: TBK
(86) International application number: PCT/JP2024/038100
(87) International publication number: WO 2025/089382

(57) **Abstract**

Provided is a photoelectric conversion element having improved conversion efficiency. The photoelectric conversion element is a photoelectric conversion element including a first electrode, a second electrode, and a photoelectric conversion layer arranged between the first electrode and the second electrode, the photoelectric conversion layer containing a crystal having a perovskite structure, wherein the photoelectric conversion element includes, between the photoelectric conversion layer and the first electrode, a charge-transporting layer containing a phthalocyanine crystal, wherein, in an X-ray diffraction spectrum of the phthalocyanine crystal using a CuKα ray, a peak is present in a range of a Bragg angle 20 of 28.0 to 29.0°, and wherein a lattice spacing d₁ [nm] calculated from a value of 20 of the peak satisfies 0.3100≤d₁≤0.3160.

## Description

### [Technical Field]

The present invention relates to a photoelectric conversion element, a photoelectric conversion apparatus, a moving body, and a building material.

### [Background Art]

In order to solve a problem of depletion of fossil energy and a global environmental problem caused by the use of fossil energy, investigations on a renewable and clean alternative energy source, such as solar energy, wind power, or water power, have been actively performed. In particular, an interest in a solar cell that directly changes sunlight into electrical energy has been increased. The term "solar cell" as used herein means a battery that generates a current-voltage utilizing a photovoltaic effect in which light energy is absorbed from sunlight to generate an electron and a hole.

Currently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem of the supply property in terms of a silicon resource.

Meanwhile, a solar cell using an organic material (hereinafter also referred to as "organic solar cell") does not require the high temperature treatment step, and is a sheet-shaped substrate in which production in a so-called roll to roll system is enabled, and hence cost reduction is expected. However, further improvements in power generation efficiency and durability have been desired for practical use of the organic solar cell. In particular, the development of a perovskite solar cell including a crystal having a perovskite structure as a photoelectric conversion layer toward the practical use of the solar cell has been advanced because the cell is excellent in photoelectric conversion property. With regard to the perovskite solar cell, it has been reported that, for example, the perovskite crystal is broken by penetration of moisture in air and migration occurs in which ions in each layer are mixed into another layer, and hence there is a problem in durability as a solar cell. In order to solve this problem, research on forming a layer for improving durability around the photoelectric conversion layer has been actively conducted.

In Patent Literature 1, there is a description of a technology for improving the photoelectric conversion efficiency and durability of a solar cell by forming a compound layer containing a compound having a phthalocyanine skeleton in a hole-transporting layer.

In Patent Literature 2, there is a description that photoelectric conversion efficiency (hereinafter also referred to as "PCE") is improved by forming a layer containing a phthalocyanine compound between a hole-transporting layer (hereinafter also referred to as "charge-transporting layer") and a perovskite. In Non Patent Literature 1, there is a description that conversion efficiency is improved by incorporating copper phthalocyanine into a hole-transporting layer.

### [Citation List]

### [Patent Literature]

PTL 1: Japanese Patent Laid-Open No. 2016-149805
PTL 2: Japanese Patent Laid-Open No. 2022-168820

### [Non Patent Literature]

NPL 1: F. Wang, et al, J. Phys. Chem. C 2017, 121, 3, 1562

### [Summary of Invention]

### [Technical Problem]

However, a further improvement in conversion efficiency toward practical use has been required to be achieved in the above-mentioned related art.

Accordingly, the present invention is directed to providing a photoelectric conversion element having improved conversion efficiency. The present invention is also directed to providing a photoelectric conversion apparatus, a moving body, and a building material each including the above-mentioned photoelectric conversion element.

### [Solution to Problem]

The above-mentioned objects are achieved by the present invention described below. That is, the present invention is directed to a photoelectric conversion element including:
a first electrode; a second electrode; and a photoelectric conversion layer arranged between the first electrode and the second electrode, the photoelectric conversion layer containing a crystal having a perovskite structure,
wherein the photoelectric conversion element includes, between the photoelectric conversion layer and the first electrode, a charge-transporting layer containing a phthalocyanine crystal,
wherein, in an X-ray diffraction spectrum of the phthalocyanine crystal using a CuKα ray, a peak is present in a range of a Bragg angle 2θ of 28.0 to 29.0°,
and wherein a lattice spacing d₁ [nm] calculated from a value of 2θ of the peak satisfies $0.3100 \leq {d}_{1} \leq 0.3160 .$

The present invention is also directed to a photoelectric conversion apparatus including the above-mentioned photoelectric conversion element.

The present invention is also directed to a moving body including the above-mentioned photoelectric conversion element.

The present invention is also directed to a building material including the above-mentioned photoelectric conversion element.

### [Advantageous Effects of Invention]

According to the present invention, the photoelectric conversion element having improved conversion efficiency can be provided.

### [Brief Description of Drawings]

[Fig. 1]

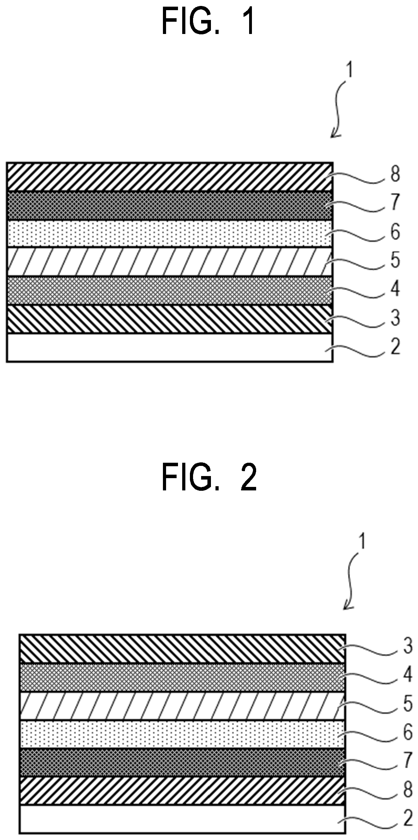

   Fig. 1 is a schematic sectional view in a thickness direction of an example of a photoelectric conversion element according to one embodiment of the present invention.
[Fig. 2]
   Fig. 2 is a schematic sectional view in a thickness direction of another example of the photoelectric conversion element according to one embodiment of the present invention.
[Fig. 3]

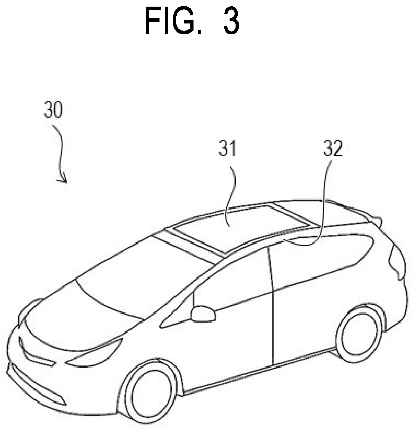

   Fig. 3 is a perspective view for schematically illustrating one embodiment of a moving body including the photoelectric conversion element of the present invention.
[Fig. 4]

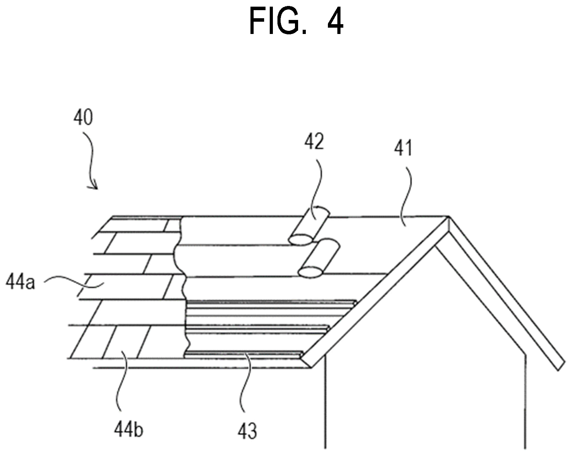

   Fig. 4 is a perspective view for schematically illustrating one embodiment of a building material including the photoelectric conversion element of the present invention.

### [Description of Embodiments]

### <One Embodiment>

One embodiment of the present invention is directed to a photoelectric conversion element.

A photoelectric conversion element of the present invention is a photoelectric conversion element including:
a first electrode; a second electrode; and a photoelectric conversion layer arranged between the first electrode and the second electrode, the photoelectric conversion layer containing a crystal having a perovskite structure,
wherein the photoelectric conversion element includes, between the photoelectric conversion layer and the first electrode, a charge-transporting layer containing a phthalocyanine crystal,
wherein, in an X-ray diffraction spectrum of the phthalocyanine crystal using a CuKα ray, a peak is present in the range of a Bragg angle 2θ of 28.0 to 29.0°,
and wherein a lattice spacing d₁ [nm] calculated from a value of 2θ of the peak satisfies $0.3100 \leq {d}_{1} \leq 0.3160 .$

According to Patent Literature 2, when a crystal having a perovskite structure is included in a photoelectric conversion layer, submicron unevenness occurs on a surface. It is estimated that interfacial joining with an electrode is stabilized by filling a recess of such unevenness with a pigment particle formed of a phthalocyanine crystal, and hence high photoelectric conversion efficiency can be obtained. However, it has been found that this alone is insufficient to obtain higher photoelectric conversion efficiency.

As a result of investigations made by the inventors of the present invention, it has been found that, in the X-ray diffraction spectrum of the phthalocyanine crystal of the present invention using a CuKα ray, a peak is present in the range of a Bragg angle 2θ of 28.0 to 29.0°, and the photoelectric conversion efficiency can be further improved when the lattice spacing d₁ [nm] calculated from the value of 2θ of the peak falls within the range of 0.3100≤d₁≤0.3160.

Details of the reason why high photoelectric conversion efficiency is obtained in the present invention are not clear, but the inventors of the present invention have presumed the reason to be as described below.

It is known that a phthalocyanine molecule has a flat plate shape, and a π-orbital thereof extends in a direction perpendicular to a molecular plane (molecular axis direction) (hereinafter also referred to as "longitudinal direction"). For this reason, a phthalocyanine crystal has a column-type structure in which planes are laminated facing each other through a π-bonding intermolecular interaction of planar molecules. In the phthalocyanine crystal, the peak that is present in the range of a Bragg angle 2θ of 28.0 to 29.0° may indicate that the phthalocyanine crystal is stacked in the longitudinal direction between a charge-generating layer and the first electrode (assuming an intermolecular distance of about 3 Å from Bragg's equation).

The stacking in the longitudinal direction is a direction in which π-electron clouds overlap as described above, and hence the transportation efficiency of a carrier that moves in a film increases because the overlap of the π-electron clouds becomes stronger as the lattice spacing d₁ becomes smaller. Thus, the photoelectric conversion efficiency is improved.

Meanwhile, it is widely known that the phthalocyanine crystal has a property of absorbing light to generate charge and is used as a material for generating a photocarrier in, for example, an electrophotographic photosensitive member. When the photoelectric conversion element of the present invention is irradiated with light, it is expected that the phthalocyanine crystal also absorbs part of the light to generate an electron pair, and the electron pair hinders transportation of a carrier from the photoelectric conversion layer toward the first electrode. When the lattice spacing d₁ is increased, the efficiency of generating the electron pair in the phthalocyanine crystal is lowered, and the efficiency of transporting the carrier is increased. Thus, the photoelectric conversion efficiency is improved.

Thorough the mechanism as described above, the inventors of the present invention have presumed that the efficiency of transporting the carrier can be increased to improve the photoelectric conversion efficiency by controlling the lattice spacing d₁ at the peak that is present in the range of a Bragg angle 2θ of 28.0 to 29.0° in the phthalocyanine crystal to an appropriate range.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which an element to be incorporated gradually changes, or a layer that may form a complicatedly intricate structure together with another layer.

Fig. 1 is a schematic sectional view for illustrating an example of the configuration of a photoelectric conversion element according to one embodiment of the present invention. A photoelectric conversion element 1 of Fig. 1 includes a substrate 2, and a second electrode 3, an electron-transporting layer 4, a photoelectric conversion layer 5, a charge-transporting layer 6, a second charge-transporting layer 7, and a first electrode 8 arranged thereon. One of the first electrode 8 and the second electrode 3 is an anode, and the other is a cathode. A current can be extracted by connecting the first electrode 8 and the second electrode 3 with an external circuit. It is preferred that the photoelectric conversion element 1 includes the second charge-transporting layer 7. When the photoelectric conversion element 1 includes the second charge-transporting layer 7, the charge-transporting layer containing a phthalocyanine crystal (charge-transporting layer 6) is a first charge-transporting layer.

The photoelectric conversion layer 5 is excited by light that has entered the layer through the substrate 2, the second electrode 3, and the electron-transporting layer 4, or the first electrode 8, the charge-transporting layer 6, and the second charge-transporting layer 7 to generate an electron or a hole. That is, the photoelectric conversion layer 5 generates a current between the first electrode 8 and the second electrode 3. The electron-transporting layer 4 is a layer arranged between the photoelectric conversion layer 5, and the two electrodes 3 and 8, and may not be formed in some cases. A form in which a plurality of the electron-transporting layers 4 and the photoelectric conversion layers 5 are laminated may be adopted. Such form may also be referred to as "tandem structure." In addition, as illustrated in Fig. 2, a configuration in which the first electrode 8, the second charge-transporting layer 7, the charge-transporting layer 6, the photoelectric conversion layer 5, the electron-transporting layer 4, and the second electrode 3 are arranged on the substrate 2 may be adopted.

The respective members are described below.

### [Photoelectric Conversion Element]

The photoelectric conversion element 1 of the present invention is the photoelectric conversion element 1 including: the first electrode 8; the second electrode 3; and the photoelectric conversion layer 5 arranged between the first electrode 8 and the second electrode 3, the photoelectric conversion layer containing a crystal having a perovskite structure, and includes the charge-transporting layer containing a phthalocyanine crystal between the photoelectric conversion layer 5 and the first electrode 8. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a crystal having a perovskite structure in the photoelectric conversion layer 5.

A method of forming each of the layers including the photoelectric conversion layer and charge-transporting layer of the photoelectric conversion element 1 of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. In Fig. 1, when light is taken in from a first electrode 8 side, an opaque material may be used as the substrate 2, and when light is taken in from a second electrode 3 side, the substrate 2 is formed of a transparent material.

### [Electrode]

A material for the first electrode 8 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof.

At least one electrode of the first electrode 8 or the second electrode 3 on a light incident side is a transparent electrode, and the other may be a transparent electrode or may also serve as a reflective layer formed of a light reflective material, or may be a transparent electrode including a reflective layer on a side opposite to the light incident side. As the photoelectric conversion element 1, it is preferred that the first electrode 8 be a positive electrode. When the first electrode 8 is on the light incident side, the second electrode 3 and the substrate 2 may be a transparent electrode and a reflective layer, respectively. The transparent electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer 5. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formula [2] to the following general formula [4].

R'₂Aₙ₋₁BₙX₃ₙ₊₁ [2]

R"Aₙ₋₁BₙX₃ₙ₊₁ [3]

R‴₂AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexadiammonium, isobutylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formula [1] to the general formula [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in the general formula [1] to the general formula [4] represents a halogen atom or the like, and examples thereof include chlorine, bromine, iodine, sulfur, and selenium. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂ (FA_{0.3}MA_{0.7}) ₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. Examples of the combinations of x1 to x5 are as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiophenethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element 1 is improved.

The crystal having a perovskite structure to be used in the present invention is preferably a crystalline semiconductor. The term "crystalline semiconductor" means a semiconductor that enables measurement of an X-ray scattering intensity distribution to detect a scattering peak. When the crystal having a perovskite structure is the crystalline semiconductor, the mobility of the electron in the crystal having a perovskite structure increases, and the photoelectric conversion efficiency of the photoelectric conversion element 1 is improved.

The thickness of the photoelectric conversion layer 5 according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

The charge-transporting layer 6 of the present invention contains a phthalocyanine crystal. In the phthalocyanine material of the present invention, a crystal means that the half-width of a peak that is present in the range of a Bragg angle 2θ of 28.0 to 29.0° in X-ray diffraction measurement to be described later is 1.0° or less.

In the photoelectric conversion element 1 of the present invention, it is preferred that the phthalocyanine crystal be a gallium phthalocyanine crystal, and it is more preferred that the phthalocyanine crystal be a hydroxygallium phthalocyanine crystal. The phthalocyanine crystal to be used in the present invention may have a center element, and examples of the center element include Ga, Cu, Ti, Zn, Si, V, Pb, Pt, Co, Sn, Mg, Fe, Al, and Mn. Of those, a gallium phthalocyanine compound having Ga as the center element is preferred. Of the gallium phthalocyanines, hydroxygallium phthalocyanine is still more preferred. The phthalocyanine crystal may be a derivative having a phthalocyanine skeleton. The gallium phthalocyanine has a satisfactory electronic interaction with a resin having a functional group of a Lewis base. Of those, hydroxygallium phthalocyanine has a strong interaction with a Lewis base by virtue of the presence of a hydroxy group, and hence it is presumed that the photoelectric conversion efficiency is improved.

Specific examples of the phthalocyanine crystal of the present invention are given below.

The phthalocyanine crystal of the present invention is, for example, hydroxygallium phthalocyanine, chlorogallium phthalocyanine, copper phthalocyanine, zinc phthalocyanine, phthalocyanine, cobalt phthalocyanine, titanyl phthalocyanine, dichlorotin phthalocyanine, magnesium phthalocyanine, tin phthalocyanine, lead phthalocyanine, iron phthalocyanine, vanadyl phthalocyanine, chloroaluminum phthalocyanine, nickel phthalocyanine, dichlorosilicon phthalocyanine, indium chlorophthalocyanine, manganese phthalocyanine, chloroiron phthalocyanine, or platinum phthalocyanine.

In addition, the phthalocyanine crystal of the present invention is, for example, naphthalocyanine, magnesium naphthalocyanine, copper naphthalocyanine, cobalt naphthalocyanine, vanadyl naphthalocyanine, tin naphthalocyanine, or dichlorotin naphthalocyanine. In the present invention, the chemical structure of the phthalocyanine crystal or the like may be identified by, for example, a nuclear magnetic resonance method (NMR).

In the photoelectric conversion element 1 of the present invention, it is preferred that the charge-transporting layer 6 contains a resin. When the content of the phthalocyanine crystal in the charge-transporting layer 6 is set to 100 parts by mass, the content of the resin in the charge-transporting layer 6 is preferably 3 to 30 parts by mass, more preferably 5 to 20 parts by mass from the viewpoints of a film-forming property and a charge-transporting ability. When the film-forming property is improved by the incorporation of the resin, the number of leak points caused by roughness of the photoelectric conversion layer 5 decreases, and hence the photoelectric conversion efficiency is improved. The content of the resin may be quantified with a nuclear magnetic resonance (NMR) apparatus or a gas chromatography apparatus.

In addition, the molecular weight of the resin is preferably 10,000 or more.

Examples of the resin to be preferably used in the present invention include a polyester resin, a polycarbonate resin, a polyvinyl acetal resin, a polyvinyl butyral resin, an acrylic resin, a polyvinyl alcohol resin, a cellulose resin, a polystyrene resin, a polyvinyl acetate resin, and a polyvinyl chloride resin.

Further, it is preferred that the resin have a glass transition temperature of 95°C or less. When the temperature falls within this range, the resin is easily brought into close contact with a charge-transporting material (charge-transporting particle), and hence a more effective charge distribution can be formed. The glass transition temperature may be determined with a differential scanning calorimeter (DSC).

The charge-transporting layer may contain an aromatic ring compound containing a hydroxy group, and the content of the aromatic ring compound containing a hydroxy group in the charge-transporting layer is preferably 1 to 30 mass%, more preferably 5 to 20 mass% with respect to the content of a cyclic conjugated compound in which a plurality of pyrrole rings are covalently bonded from the viewpoints of the film-forming property and the charge-transporting ability.

An example of the aromatic ring compound containing a hydroxy group to be preferably used in the present invention is a calixarene compound.

In addition, in the photoelectric conversion element 1 of the present invention, in an X-ray diffraction spectrum using a CuKα ray for the charge-transporting layer 6 having a phthalocyanine crystal, a peak is present in the range of a Bragg angle 2θ of 28.0 to 29.0°, and a lattice spacing d₁ [nm] calculated from a value of 2θ of the peak satisfies 0.3100≤d₁≤0.3160. In the photoelectric conversion element 1 of the present invention, the lattice spacing d₁ [nm] calculated from the value of 2θ of the peak preferably satisfies 0.3120≤d₁≤0.3160, and more preferably satisfies 0.3145≤d₁≤0.3155.

In the present invention, in the X-ray diffraction spectrum using a CuKα ray, the lattice spacing d₁ [nm] calculated from the value of 2θ of the peak that is present in the range of a Bragg angle 2θ of 28.0 to 29.0° may be calculated by using Bragg's equation that is the following equation (1) using θ₁ [rad], which is θ obtained from the peak that is present in the range of 28.0 to 29.0°, and an X-ray wavelength λ [nm] (λ=0.15418 in the case of the X-ray diffraction spectrum using a CuKα ray). ${d}_{1} = λ / \left(2sin{θ}_{1}\right)$

Here, when a peak derived from a phthalocyanine crystal is present in the range of 28.0 to 29.0°, an angle θ at a point having the highest peak intensity is defined as θ₁.

Here, the meanings of the terms "particle," "crystallite," and "crystallite size d₂ (nm)" of a phthalocyanine crystal in the present invention are described. In the present invention, the term "particle" of the phthalocyanine crystal is a primary particle of the phthalocyanine crystal in which phthalocyanine molecules are aggregated and integrated. Meanwhile, the term "crystallite" of the phthalocyanine crystal is a part of the minimum unit that can be regarded as a single crystal of phthalocyanine in the above-mentioned crystal particle, and the crystallite size d₂ means a size of the crystallite.

In the present invention, the crystallite size d₂ [nm] of the phthalocyanine crystal was calculated from a peak that is present in the range of a Bragg angle 2θ of 28.0 to 29.0° in an X-ray diffraction spectrum using a CuKα ray. The crystallite size d₂ may be represented by the following equation (2) using a Scherrer constant K [-], an X-ray wavelength λ [nm], a half-width β [rad], and θ₁ [rad] from Scherrer's equation: ${d}_{2} = K ⋅ λ / \left(β⋅cos{θ}_{1}\right)$

As the crystallite size d₂ becomes larger, the number of interfaces of crystallites, which cause inhibition of transportation of a carrier injected from the photoelectric conversion layer 5, decreases, and hence a resistance increase and a voltage drop due to stagnation of a carrier are suppressed, and the photoelectric conversion efficiency is improved. In the photoelectric conversion element 1 of the present invention, the crystallite size d₂ calculated from the half-width of the peak preferably satisfies d₂≥19.5, and more preferably satisfies d₂≥20.0.

Meanwhile, when the crystallite is too large, variation occurs in crystal size and the surface property of the charge-transporting layer 6 deteriorates, and hence the shunt resistance decreases and the photoelectric conversion efficiency is adversely affected. In the photoelectric conversion element 1 of the present invention, the crystallite size d₂ calculated from the half-width of the peak preferably satisfies d₂≤21.1.

The number-average particle diameter of the phthalocyanine crystal is preferably 50 to 150 nm.

Examples of a dispersion method for forming the phthalocyanine crystal into particles include methods using a paint shaker, a sand mill, a ball mill, and a liquid-collision type high-speed disperser. Values of d₁ and d₂ are significantly different because a load applied to a phthalocyanine material is different when a dispersion system is changed.

Of those, a sand mill forms a crystal into a particle by rotation of a disc rotating in a mill and a shearing force by a medium such as glass beads serving as a grinding medium. At that time, the lattice spacing d₁, the crystallite size d₂, and the number-average particle diameter change because crystallinity changes depending on dispersion conditions, such as a dispersion time, an amount of beads, and the number of rotations of the disc. For example, both d₁ and d₂ tend to decrease when the dispersion time is extended to an extent that over-dispersion (e.g., aggregation of particles or formation of fragments) does not occur. In addition, it is possible to change d₂ while suppressing a change in d₁ to be small by changing a temperature during dispersion. It is presumed that the change amount of each of d₁ and d₂ changes based on a balance between a change in promoting the growth of crystallites by increasing the temperature and a change in dispersion intensity caused by a temperature change.

In addition, d₁ and d₂ significantly differ depending on the kind of the center element of the phthalocyanine crystal, a crystal form, or a solvent during dispersion.

The phthalocyanine crystal may contain part of the solvent used for dispersion in the crystal. The solvent in the crystal is preferably any one selected from the group consisting of: N-methylformamide; N-ethylformamide; N-propylformamide; and dimethyl sulfoxide. Of those, N-methylformamide is still more preferred. That is, in the photoelectric conversion element 1 of the present invention, it is preferred that the phthalocyanine crystal contains N-methylformamide. The mobility of the carrier increases and the photoelectric conversion efficiency is improved because those materials each have a large pKa and a large dipole moment, and thus charge imbalance inside the material becomes large. A method of introducing the organic compound into the crystal is not particularly limited, and for example, there is given a method of causing the organic compound to coexist in a process of crystallization. As an amount of the organic compound in the crystal, there is given a method of changing various conditions, such as a treatment time and a treatment intensity, in the process of crystallization .

It is conceived that, when the roughness of the photoelectric conversion layer 5 is large, the photoelectric conversion layer 5 and the first electrode 8 are brought into direct contact with each other, causing a decrease in photoelectric conversion efficiency along with a decrease in shunt resistance and a decrease in durability during long-term storage. For that reason, it becomes possible to suppress contact between the photoelectric conversion layer 5 and the first electrode 8 and improve the photoelectric conversion efficiency and durability by increasing the thickness of the charge-transporting layer 6 to a certain level or more. Meanwhile, the carrier transportation efficiency decreases because when the thickness of the charge-transporting layer 6 is too large, and hence it is preferred that the thickness of the charge-transporting layer 6 be prevented from being too large. In the photoelectric conversion element 1 of the present invention, the average thickness of the charge-transporting layer 6 is preferably 30 to 400 nm, more preferably 50 to 180 nm.

Measurement of an X-ray diffraction spectrum of the charge-transporting layer 6 and observation of the incorporated phthalocyanine crystal may be performed by, for example, exposing a surface of the charge-transporting layer 6 after removing a layer above the charge-transporting layer 6 of the photoelectric conversion element 1 with an organic solvent such as chloroform. In Examples of the present invention, unless otherwise specified, the X-ray diffraction spectrum was measured and the incorporated phthalocyanine crystal was observed under a state in which the charge-transporting layer 6 was exposed by the above-mentioned method.

### [X-ray Diffraction Measurement]

In the present invention, an X-ray diffraction spectrum of the exposed charge-transporting layer 6 was measured under the following conditions. The resultant X-ray diffraction spectrum of the charge-transporting layer 6 was subjected to smoothing treatment, a peak position in a peak that is present in the range of 28.0 to 29.0° was defined as θ₁ [rad], and a half-width in the peak was defined as a half-width β [rad]. In addition, in analysis, a Scherrer constant K differs depending on a shape of a crystal and a way of taking a half-width, but in the present invention, calculation was performed with K=0.89.
Measuring instrument used: X-ray diffractometer RINT-TTRII manufactured by Rigaku Corporation
X-ray tube: Cu
X-ray wavelength: Kα1
Tube voltage: 50 KV
Tube current: 300 mA
Scanning method: 2θ-θ scan
Scanning speed: 0.5°/min
Sampling interval: 0.01°
Start angle 2θ: 5.0°
Stop angle 2θ: 35.0°
Goniometer: Rotor horizontal goniometer (TTR-2)
Filter: None
Detector: Scintillation counter
Incident monochromator: Used
Slit: Variable slit (Parallel beam method)
Counter monochromator: Not used
Divergence slit: Open
Divergence longitudinal limiting slit: 10.00 mm
Scattering slit: Open
Receiving slit: Open

### [Analysis of Compound Amount]

In Examples of the present invention, a surface of the charge-transporting layer 6 of the photoelectric conversion element 1 was wiped with a cotton swab or the like to which a solvent was applied, and was dissolved in heavy sulfuric acid, and ¹H-NMR measurement (Apparatus: AVANCE 3-500 manufactured by BRUKER) was performed. In addition, the component that had been stripped off was subjected to mass and structural analysis, such as GPC and MALDI-TOF-MS, IR, and gas chromatography, and elemental analysis, such as EDX and XPS, and the kind of the phthalocyanine crystal, and the presence of an organic compound in the crystal such as N-methylformamide or a compound such as a resin was determined together with the results of the above-mentioned X-ray diffraction measurement.

### [Measurement of Average Thickness]

In the present invention, the average thickness of the charge-transporting layer 6 was determined by sectional observation using a scanning electron microscope (hereinafter also referred to as "SEM") (apparatus: SmartSEM, Carl Zeiss Co., Ltd.) after the photoelectric conversion element 1 was cut and fixed to an inclined sample stage. In the observation, the charge-transporting layer 6 was distinguished from other layers based on a difference in contrast of an observed image and composition analysis by a SEM-EDX function, and an average value of thicknesses of a portion of the charge-transporting layer 6 was measured by image processing from an image taken at a magnification of 50,000. This was taken at five random locations, and an average of the five average values was defined as the average thickness of the charge-transporting layer 6.

### [Second Charge-transporting Layer]

It is preferred that the photoelectric conversion element 1 of the present invention include the second charge-transporting layer 7 between the charge-transporting layer 6 and the first electrode 8 from the viewpoint of the compatibility of a film of the charge-transporting layer. In that case, the charge-transporting layer containing a phthalocyanine crystal (charge-transporting layer 6) is a first charge-transporting layer.

A material for the second charge-transporting layer 7 is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound particularly preferably has an aromatic ring from the viewpoint of the compatibility of a film interface, and Spiro-OMeTA or PTAA is more preferred.

In addition, the second charge-transporting layer may contain a dopant as an additive in order to improve charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element 1 of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type lowmolecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 4 is 1 nm, and a preferred upper limit thereof is 2,000 nm. When the thickness of the electron-transporting layer 4 is 1 nm or more, a hole can be sufficiently blocked, and when the thickness is 2,000 nm or less, the electron-transporting layer 4 is less liable to serve as resistance at the time of the electron transportation, and hence the photoelectric conversion efficiency increases. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus of the present invention includes the above-mentioned photoelectric conversion element. The photoelectric conversion apparatus may be formed by using a plurality of photoelectric conversion elements of the present invention. When a plurality of photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function, such as maintaining or increasing an amount of incident light, a surface layer to which water or dirt is hard to adhere, or a function of collecting or guiding light may be added.

### [Moving Body]

A moving body of the present invention includes the above-mentioned photoelectric conversion element. Fig. 3 is a perspective view for schematically illustrating one embodiment of a moving body including the photoelectric conversion element of the present invention. A moving body 30 includes a photoelectric conversion element 31 of the present invention and a body 32 including the photoelectric conversion element 31. The photoelectric conversion element 31 is arranged on a position of the body 32 at which ambient light can be received. When the moving body 30 is an automobile, the photoelectric conversion element 31 may be arranged on a roof. Electric energy obtained by the photoelectric conversion element 31 may serve as power of the moving body 30 or power of any other electric equipment. Electric energy generated from the power of the moving body 30 may be used for power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the above-mentioned photoelectric conversion element. Fig. 4 is a perspective view for schematically illustrating one embodiment of a building material including the photoelectric conversion element of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be reduced by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, may be the same. The exterior 44a and the exterior 44b may be formed of the same member or may be formed of different members. A paint or a transparent substrate may be used as the exterior. An exterior having small light absorption and a high heat-shielding property is preferred.

In addition to the application examples described above, the following application examples may be given: a portable device, such as a calculator, a sensor, and a small solar panel; a wearable device, such as a glasses-type terminal, a watchtype terminal, and a portable medical device; sheet structures supported by a plurality of frames, such as a tent, a plastic house, and a loading platform of a truck; and structures to be used by being fixed, such as a road surface panel, a floating panel, a building material utilizing flexibility of a substrate, a wall-type building material, a glass-type building material, and a mega solar panel.

### [With regard to Method of producing Photoelectric Conversion Element]

A method of producing a photoelectric conversion element of the present invention includes a step of forming a first electrode, a step of forming a second electrode, a step of forming a photoelectric conversion layer containing a crystal having a perovskite structure between the first electrode and the second electrode, and a step of forming a charge-transporting layer between the photoelectric conversion layer and the first electrode.

The respective steps of the manufacturing method are described below.

### (Step of forming First Electrode and Step of forming Second Electrode)

A method of producing a photoelectric conversion element of the present invention includes a step of forming a first electrode and a step of forming a second electrode. In the step of forming the first electrode and the step of forming the second electrode, an appropriate method may be selected in accordance with a material of the first electrode and a material of the second electrode, respectively. Examples of such method include, but are not limited to, a sputtering method, a vacuum vapor deposition method, a vapor phase growth method (CVD method), and a spray pyrolysis deposition method (SPD method). Materials of the first electrode and the second electrode are as described above. When one or both of the first electrode and the second electrode are transparent electrodes, the thickness of the transparent electrode is preferably 0.03 to 3 µm.

When a solar cell is produced, cutting processing may be performed for circuit formation between steps. Examples of the cutting processing include mechanical patterning and laser patterning.

### (Modularization Step)

An element formed up to the electrode may be sealed. Examples of a sealing method include sealing with a resin or sealing with a film. Examples of a material used for sealing include silazane, silicone rubber, resins having a siloxane skeleton, and glass.

In addition, a hairline treatment may be applied to a surface of the sealed element from the viewpoint of suppression of adhesion between elements occurring during winding in a roll-to-roll system.

### (Step of forming Photoelectric Conversion Layer)

A method of producing a photoelectric conversion element of the present invention includes a step of forming a photoelectric conversion layer containing a crystal having a perovskite structure between the first electrode and the second electrode. The step of forming the photoelectric conversion layer may include a step of applying a liquid containing the material of the photoelectric conversion layer as described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with properties of a photoelectric conversion layer to be produced, such as thickness control and orientation control.

An annealing treatment may be performed under reduced pressure or in an inert atmosphere (in a nitrogen or argon atmosphere) in order to remove a solvent or a dispersion medium from the liquid containing the applied material of the photoelectric conversion layer. The temperature of the annealing treatment is preferably 40 to 300°C, more preferably 50 to 150°C. The annealing treatment is preferably performed because materials for forming each layer may permeate each other at an interface between laminated layers to increase a contact area, and hence a short-circuit current can be increased.

### (Step of forming Charge-transporting Layer)

As a step of forming the charge-transporting layer, a method of applying a liquid containing the material of the charge-transporting layer as described above is preferred. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method.

In addition, examples of the step of forming the charge-transporting layer include: a method including arranging a charge-transporting particle on a surface of a photoelectric conversion layer, and then applying and drying a resin solution in which a resin is dissolved; a method including applying a resin solution in which a resin is dissolved on a surface of a photoelectric conversion layer, arranging a charge-transporting particle, and then drying the resin solution; and a method including applying a solution in which charge-transporting particle is dispersed in a resin solution in which a resin is dissolved on a surface of a photoelectric conversion layer, and drying the solution.

### Examples

The present invention is described in more detail below by way of Examples and Comparative Examples. The present invention is by no means limited to the following Examples without departing from the gist thereof. In the description of the following Examples, "part(s)" is by mass unless otherwise specified.

### (Step of producing Phthalocyanine Crystal 1)

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71 mass%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine particle (crystal) having a water content of 1.0 mass% or less was obtained.

### Step (3)

5 Parts of the hydroxygallium phthalocyanine particle was subjected to dispersion treatment at 25°C for 30 hours with a sand mill (TSG-1/4G-4U, manufactured by Igarashi Machine Production Co., Ltd. (currently AIMEX Co., Ltd.), disc diameter: 70 mm, number of discs: 5) loaded with 100 parts of an N-methylformamide solvent and 100 parts of glass beads while discs were rotated at 300 rpm. The resultant was filtered and dried to provide a phthalocyanine crystal 1.

### (Steps of producing Phthalocyanine Crystals 2 to 16)

Phthalocyanine crystals 2 to 16 were each produced in the same manner as in the production of the phthalocyanine crystal 1 except that, in the production of the phthalocyanine crystal 1, the solvent used in Step (3), and the temperature and the dispersion time during the dispersion treatment were changed as shown in Table 2. The phthalocyanine crystal 14 was produced by performing the dispersion, the filtration, and the drying in Step (3), followed by centrifugal separation to remove coarse particles.

### [Table 2]

**Table 2**

| Phthalocyanine crystal | Dispersion solvent | Dispersion temperature [°C] | Dispersion time [h] | Others |
|---|---|---|---|---|
| Phthalocyanine crystal 1 | N-Methylformamide | 25 | 30 | - |
| Phthalocyanine crystal 2 | N-Methylformamide | 25 | 100 | - |
| Phthalocyanine crystal 3 | N-Methylformamide | 25 | 5 | - |
| Phthalocyanine crystal 4 | N-Methylformamide | 25 | 2 | - |
| Phthalocyanine crystal 5 | N-Propylformamide | 25 | 30 | - |
| Phthalocyanine crystal 6 | Formamide | 25 | 70 | - |
| Phthalocyanine crystal 7 | N-Methylformamide | 40 | 30 | - |
| Phthalocyanine crystal 8 | N-Methylformamide | 45 | 30 | - |
| Phthalocyanine crystal 9 | N-Methylformamide | 35 | 30 | - |
| Phthalocyanine crystal 10 | N-Methylformamide | 20 | 30 | - |
| Phthalocyanine crystal 11 | N,N-Dimethylformamide | 25 | 5 | - |
| Phthalocyanine crystal 12 | N,N-Dimethylformamide | 25 | 10 | - |
| Phthalocyanine crystal 13 | N,N-Dimethylformamide | 25 | 15 | - |
| Phthalocyanine crystal 14 | N-Methylformamide | 20 | 30 | Centrifugal separation was performed |
| Phthalocyanine crystal 15 | N-Propylformamide | 25 | 50 | - |
| Phthalocyanine crystal 16 | Formamide | 25 | 25 | - |

### (Production of Resin Solution 1)

1.0 Gram of polyvinyl butyral (product name: BM-2, manufactured by Sekisui Chemical Co., Ltd., glass transition temperature: 71°C) was dissolved in 19 g of 2-propanol by stirring for 24 hours to provide a resin solution 1.

### (Production of Resin Solution 2)

1.0 Gram of polyacrylic acid (molecular weight: 5,000, manufactured by FUJIFILM Wako Pure Chemical Corporation) was dissolved in 19 g of ethanol by stirring for 24 hours to provide a resin solution 2.

### (Production of Resin Solution 3)

1.0 Gram of polymethyl methacrylate (PMMA, manufactured by Sigma-Aldrich Co. LLC, glass transition temperature: 70°C) was dissolved in 19 g of 2-propanol by stirring for 24 hours to provide a resin solution 3.

### (Production of Resin Solution 4)

1.0 Gram of polymethyl methacrylate (PMMA, manufactured by Sigma-Aldrich Co. LLC, glass transition temperature: 100°C) was dissolved in 19 g of 2-propanol by stirring for 24 hours to provide a resin solution 4.

### (Example 1)

### [Formation of Electron-transporting Layer]

A glass substrate with ITO was washed, and tin(II) oxide prepared to 3 mass% was applied thereonto by spin coating. After that, the resultant was heated at 150°C for 30 minutes to form an electron-transporting layer as a thin film having a thickness of 15 nm.

### [Formation of Photoelectric Conversion Layer]

22.4 Milligrams of methylammonium bromide, 172 mg of formamidinium iodide, and 576 mg of lead iodide were dissolved in 500 µL of N,N-dimethylformamide and 200 µL of dimethyl sulfoxide, and were stirred for 1 hour (solution 1). Further, 389.72 mg of cesium iodide was dissolved in 1,000 µL of dimethyl sulfoxide and was stirred for 1 hour (solution 2). After that, 40 µL of the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.96}Pb(I_{0.95}Br_{0.05})₃ and having a thickness of 500 nm.

### [Formation of Charge-transporting Layer]

0.1 Gram of the phthalocyanine crystal 1 and 0.01 g of a calixarene compound (Japanese Patent Laid-Open No. 2003-207913) were dispersed for 6 hours with a paint shaker (manufactured by Toyo Seiki Seisaku-sho, Ltd.) loaded with 10.6 g of 2-propanol and 11 g of zirconia beads. After that, 0.2 g of the resin solution 1 was added thereto, followed by paint shaker dispersion for 6 hours again to prepare a coating liquid for a charge-transporting layer. The resultant coating liquid for a charge-transporting layer was applied onto the photoelectric conversion layer by spin coating to form a charge-transporting layer having a thickness of 100 nm.

### [Formation of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithiumbis(trifluoromethanesulfonyl)imide in 0.3 g of acetonitrile and 36 µL of t-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.5 g of acetonitrile was mixed thereinto to prepare a coating liquid 1 for a charge-transporting layer. The coating liquid 1 for a charge-transporting layer was applied onto the charge-transporting layer by a spin coating method to form a second charge-transporting layer having a thickness of 100 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

The resultant photoelectric conversion element was subjected to the above-mentioned measurement of compound amounts, measurement of N-methylformamide, measurement of an average thickness, and X-ray diffraction measurement to determine the composition of each of the phthalocyanine crystals, the presence or absence of N-methylformamide (NMF) in the phthalocyanine crystal, and the thickness of the charge-transporting layer, and to calculate d₁ and d₂. The results are shown in Tables 3 and 4.

### (Production of Examples 2 and 3)

Examples 2 and 3 were each produced in the same manner as in the production of Example 1 except that, in the production of Example 1, the kind of the phthalocyanine crystal to be used and the loading amount of the phthalocyanine crystal were changed as shown in Table 3.

### (Production of Examples 4 to 28)

Examples 4 to 28 are each produced in the same manner as in the production of Example 1 except that, in the production of Example 1, the kind of the phthalocyanine crystal to be used, the loading amount of the phthalocyanine crystal, and the kind of the resin solution are changed as shown in Table 3. However, in each of Examples 14 to 16, in the formation of the charge-transporting layer, the coating liquid for a charge-transporting layer is prepared without addition of the resin solution and subsequent paint shaker dispersion. In addition, in Example 26, Example 26 is produced without formation of the second charge-transporting layer.

### [Table 3]

**Table 3**

| Example | First charge-transporting layer | | | | | | |
|---|---|---|---|---|---|---|---|
| | Phthalocyanine crystal | | | | Resin solution | Presence or absence of resin | Average thickness [nm] |
| | Kind | Center metal | Loading amount [g] | Presence or absence of NMF | | | |
| Example 1 | Phthalocyanine crystal 1 | Hydroxygallium | 0.1 | Present | Resin solution 1 | Present | 100 |
| Example 2 | Phthalocyanine crystal 2 | Hydroxygallium | 0.1 | Present | Resin solution 1 | Present | 100 |
| Example 3 | Phthalocyanine crystal 3 | Hydroxygallium | 0.1 | Present | Resin solution 1 | Present | 100 |
| Example 4 | Phthalocyanine crystal 4 | Hydroxygallium | 0.1 | Present | Resin solution 1 | Present | 100 |
| Example 5 | Phthalocyanine crystal 5 | Hydroxygallium | 0.1 | Absent | Resin solution 1 | Present | 100 |
| Example 6 | Phthalocyanine crystal 6 | Hydroxygallium | 0.1 | Absent | Resin solution 1 | Present | 100 |
| Example 7 | Phthalocyanine crystal 7 | Hydroxygallium | 0.1 | Present | Resin solution 1 | Present | 100 |
| Example 8 | Phthalocyanine crystal 8 | Hydroxygallium | 0.1 | Present | Resin solution 1 | Present | 100 |
| Example 9 | Phthalocyanine crystal 9 | Hydroxygallium | 0.1 | Absent | Resin solution 1 | Present | 100 |
| Example 10 | Phthalocyanine crystal 10 | Hydroxygallium | 0.1 | Absent | Resin solution 1 | Present | 100 |
| Example 11 | Phthalocyanine crystal 11 | Hydroxygallium | 0.1 | Absent | Resin solution 1 | Present | 100 |
| Example 12 | Phthalocyanine crystal 12 | Hydroxygallium | 0.1 | Present | Resin solution 1 | Present | 100 |
| Example 13 | Phthalocyanine crystal 13 | Hydroxygallium | 0.1 | Present | Resin solution 1 | Present | 100 |
| Example 14 | Phthalocyanine crystal 1 | Hydroxygallium | 0.1 | Present | - | Present | 100 |
| Example 15 | Phthalocyanine crystal 2 | Hydroxygallium | 0.1 | Present | - | Present | 100 |
| Example 16 | Phthalocyanine crystal 3 | Hydroxygallium | 0.1 | Present | - | Present | 100 |
| Example 17 | Phthalocyanine crystal 1 | Hydroxygallium | 0.1 | Present | Resin solution 2 | Present | 100 |
| Example 18 | Chlorogallium phthalocyanine 1 | Chlorogallium | 0.1 | Absent | Resin solution 1 | Present | 100 |
| Example 19 | Vanadyl phthalocyanine | Vanadium | 0.1 | Absent | Resin solution 1 | Present | 100 |
| Example 20 | Copper phthalocyanine 1 | Copper | 0.1 | Absent | Resin solution 1 | Present | 100 |
| Example 21 | Titanyl phthalocyanine | Titanium | 0.1 | Absent | Resin solution 1 | Present | 100 |
| Example 22 | Phthalocyanine crystal 1 | Hydroxygallium | 0.4 | Present | Resin solution 1 | Present | 180 |
| Example 23 | Phthalocyanine crystal 1 | Hydroxygallium | 0.5 | Present | Resin solution 1 | Present | 300 |
| Example 24 | Phthalocyanine crystal 14 | Hydroxygallium | 0.08 | Present | Resin solution 1 | Present | 50 |
| Example 25 | Phthalocyanine crystal 14 | Hydroxygallium | 0.05 | Present | Resin solution 1 | Present | 30 |
| Example 26 | Phthalocyanine crystal 1 | Hydroxygallium | 0.1 | Present | Resin solution 1 | Present | 100 |
| Example 27 | Phthalocyanine crystal 1 | Hydroxygallium | 0.1 | Present | Resin solution 3 | Present | 100 |
| Example 28 | Phthalocyanine crystal 1 | Hydroxygallium | 0.1 | Present | Resin solution 4 | Present | 100 |
| Comparative Example 1 | Hydroxygallium phthalocyanine | Hydroxygallium | 0.1 | Absent | - | Present | 200 |
| Comparative Example 2 | Hydroxygallium phthalocyanine | Hydroxygallium | 0.1 | Absent | - | Present | 700 |
| Comparative Example 3 | Chlorogallium phthalocyanine 2 | Chlorogallium | 0.1 | Absent | Resin solution 1 | Present | 100 |
| Comparative Example 4 | Copper phthalocyanine 2 | Copper | 0.1 | Absent | Resin solution 1 | Present | 100 |
| Comparative Example 5 | Phthalocyanine crystal 15 | Hydroxygallium | 0.1 | Absent | Resin solution 1 | Present | 100 |
| Comparative Example 6 | Phthalocyanine crystal 16 | Hydroxygallium | 0.1 | Absent | Resin solution 1 | Present | 100 |

### (Production of Comparative Example 1)

Comparative Example 1 was produced in the same manner as in Example 1 except that, in the production of Example 1, the charge-transporting layer was changed to the particle layer produced in Example 1 described in Japanese Patent Laid-Open No. 2022-168820.

### (Production of Comparative Example 2)

Comparative Example 1 was produced in the same manner as in Example 1 except that, in the production of Example 1, the charge-transporting layer was changed to the particle layer produced in Example 9 described in Japanese Patent Laid-Open No. 2022-168820.

### (Production of Comparative Example 3)

Comparative Example 3 is produced in the same manner as in the production of Example 18 except that, in the production of Example 18, the chlorogallium phthalocyanine 1 was changed to chlorogallium phthalocyanine 2 having strong peaks at 9.0±0.2°, 17.6±0.2°, 27.4±0.2°, and 28.8±0.2° in an X-ray diffraction spectrum.

### (Production of Comparative Example 4)

Comparative Example 4 is produced in the same manner as in the production of Example 20 except that, in the production of Example 20, the copper phthalocyanine 1 was changed to copper phthalocyanine 2 having strong peaks at 7.0±0.2°, 9.2±0.2°, 12.5±0.2°, 16.8±0.2°, 18.6±0.2°, 21.3±0.2°, 23.8±0.2°, 26.2±0.2°, 28.0±0.2°, and 30.5±0.2° in an X-ray diffraction spectrum.

### (Production of Comparative Examples 5 and 6)

Comparative Examples 5 and 6 are each produced in the same manner as in the production of Example 1 except that, in the production of Example 1, the phthalocyanine crystal 1 was changed as shown in Table 3.

### [Evaluation]

### [Photoelectric Conversion Efficiency Evaluation]

A power source (manufactured by Keithley Instruments, Model 236) was connected between the electrodes of each of the photoelectric conversion elements, and constant light was applied with a solar simulator (manufactured by Yamashita Denso Corporation) at an intensity of 100 mW/cm², and a current and a voltage to be generated were measured. Thus, photoelectric conversion efficiency was evaluated. A series resistance was calculated by approximating the reciprocal of a slope near the open circuit voltage (Voc) of the resultant current-voltage curve, and a shunt resistance was calculated by approximating the reciprocal of a slope near the short-circuit current density (Jsc) of the resultant current-voltage curve. The results are shown in Table 4.

### [X-ray Diffraction Measurement]

The sample obtained in Example 1 was subjected to X-ray diffraction measurement by the above-mentioned method. In the measurement, a sample in which a layer above the charge-transporting layer was peeled off and a sample in which up to the charge-transporting layer was peeled off were prepared, and an X-ray diffraction spectrum of only the charge-transporting layer was acquired by taking a difference between the two data, and values of 2θ and a half-width were calculated.

Subsequently, X-ray diffraction measurement was performed by preparing a sample in which up to the photoelectric conversion layer was formed and a sample in which up to the charge-transporting layer was formed in the production of Example 1. As a result, values of 2θ and a full width at half maximum were the same as the results of X-ray diffraction of the sample obtained by peeling off the upper layer. The evaluation of the samples produced in Examples 2 to 28 and Comparative Examples 1 to 6 is performed by using a sample in which up to the photoelectric conversion layer is formed and a sample in which up to the charge-transporting layer is formed. The results are shown in Table 4.

### [Analysis of Compound Amounts and Measurement of Average Thickness]

The kind of the compound of each of the phthalocyanine crystals, the presence or absence of NMF in the phthalocyanine crystal, the average thickness of the charge-transporting layer, and the presence of a resin in the charge-transporting layer were determined by the above-mentioned methods. The results are shown in Table 3.

### [Table 4]

**Table 4**

| Example | First charge-transporting layer | | | | Element characteristics | | |
|---|---|---|---|---|---|---|---|
| | X-ray diffraction 28.0 to 29.0° peak | | | | Photoelectric conversion efficiency [%] | Series resistance [Ω] | Shunt resistance [Ω] |
| | 2θ [°] | d₁ [nm] | Half-width [°] | d₂ [nm] | | | |
| Example 1 | 28.34 | 0.3146 | 0.390 | 20.8 | 18.5 | 43 | 9,517 |
| Example 2 | 28.38 | 0.3142 | 0.410 | 19.8 | 17.2 | 68 | 8,033 |
| Example 3 | 28.26 | 0.3155 | 0.379 | 21.4 | 16.9 | 43 | 6,402 |
| Example 4 | 28.22 | 0.3160 | 0.370 | 21.9 | 15.8 | 82 | 6,733 |
| Example 5 | 28.76 | 0.3101 | 0.390 | 20.8 | 15.8 | 85 | 7,726 |
| Example 6 | 28.22 | 0.3160 | 0.390 | 20.8 | 15.5 | 83 | 7,618 |
| Example 7 | 28.34 | 0.3146 | 0.414 | 19.6 | 17.1 | 66 | 8,025 |
| Example 8 | 28.34 | 0.3146 | 0.420 | 19.3 | 16.1 | 104 | 8,219 |
| Example 9 | 28.34 | 0.3146 | 0.405 | 20.0 | 18.2 | 54 | 7,647 |
| Example 10 | 28.34 | 0.3146 | 0.384 | 21.1 | 17.9 | 44 | 7,510 |
| Example 11 | 28.40 | 0.3140 | 0.407 | 19.9 | 16.8 | 84 | 8,391 |
| Example 12 | 28.54 | 0.3125 | 0.412 | 19.7 | 15.3 | 118 | 8,436 |
| Example 13 | 28.60 | 0.3118 | 0.425 | 19.1 | 14.8 | 150 | 8,572 |
| Example 14 | 28.33 | 0.3148 | 0.390 | 20.8 | 16.5 | 41 | 5,285 |
| Example 15 | 28.37 | 0.3143 | 0.410 | 19.8 | 15.2 | 72 | 5,327 |
| Example 16 | 28.25 | 0.3156 | 0.381 | 21.3 | 14.3 | 84 | 3,427 |
| Example 17 | 28.34 | 0.3146 | 0.390 | 20.8 | 18.2 | 44 | 7,731 |
| Example 18 | 28.76 | 0.3101 | 0.430 | 18.9 | 14.1 | 194 | 7,996 |
| Example 19 | 28.56 | 0.3123 | 0.447 | 18.1 | 13.5 | 270 | 7,832 |
| Example 20 | 28.22 | 0.3160 | 0.471 | 17.2 | 13.1 | 325 | 8,157 |
| Example 21 | 28.62 | 0.3116 | 0.465 | 17.4 | 12.9 | 396 | 8,123 |
| Example 22 | 28.34 | 0.3146 | 0.390 | 20.8 | 17.7 | 50 | 7,658 |
| Example 23 | 28.34 | 0.3146 | 0.390 | 20.8 | 16.3 | 122 | 9,730 |
| Example 24 | 28.34 | 0.3146 | 0.390 | 20.8 | 18.0 | 39 | 7,115 |
| Example 25 | 28.34 | 0.3146 | 0.390 | 20.8 | 16.7 | 36 | 4,784 |
| Example 26 | 28.34 | 0.3146 | 0.390 | 20.8 | 12.5 | 356 | 3,225 |
| Example 27 | 28.34 | 0.3146 | 0.390 | 20.8 | 17.6 | 70 | 9,687 |
| Example 28 | 28.34 | 0.3146 | 0.390 | 20.8 | 16.4 | 102 | 9,821 |
| Comparative Example 1 | 28.17 | 0.3165 | 0.410 | 19.8 | 10.7 | 769 | 7,436 |
| Comparative Example 2 | 28.92 | 0.3085 | 0.425 | 19.1 | 10.3 | 782 | 5,227 |
| Comparative Example 3 | 28.90 | 0.3087 | 0.467 | 17.4 | 9.4 | 881 | 7,372 |
| Comparative Example 4 | 28.00 | 0.3184 | 0.454 | 17.8 | 9.1 | 923 | 7,114 |
| Comparative Example 5 | 28.92 | 0.3085 | 0.442 | 18.4 | 10.0 | 802 | 7,038 |
| Comparative Example 6 | 28.08 | 0.3175 | 0.380 | 21.3 | 8.2 | 974 | 6,523 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, and Japanese Patent Application No. 2024-086010 filed on May 28, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 second charge-transporting layer
8 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises, between the photoelectric conversion layer and the first electrode, a charge-transporting layer containing a phthalocyanine crystal,
wherein, in an X-ray diffraction spectrum of the phthalocyanine crystal using a CuKα ray, a peak is present in a range of a Bragg angle 2θ of 28.0 to 29.0°, and
wherein a lattice spacing d₁ [nm] calculated from a value of 2θ of the peak satisfies 0.3100≤d₁≤0.3160.

2. The photoelectric conversion element according to claim 1, wherein a crystallite size d₂ [nm] calculated from a half-width of the peak satisfies d₂≥19.5.

3. The photoelectric conversion element according to claim 1, wherein a crystallite size d₂ [nm] calculated from a half-width of the peak satisfies d₂≥20.0.

4. The photoelectric conversion element according to any one of claims 1 to 3, wherein a crystallite size d₂ [nm] calculated from a half-width of the peak satisfies d₂≤21.1.

5. The photoelectric conversion element according to any one of claims 1 to 4, wherein the lattice spacing d₁ [nm] satisfies 0.3145≤d₁≤0.3155.

6. The photoelectric conversion element according to any one of claims 1 to 5, wherein the charge-transporting layer contains a resin.

7. The photoelectric conversion element according to any one of claims 1 to 6, further comprising a second charge-transporting layer between the first electrode and the charge-transporting layer.

8. The photoelectric conversion element according to any one of claims 1 to 7, wherein the phthalocyanine crystal is a gallium phthalocyanine crystal.

9. The photoelectric conversion element according to any one of claims 1 to 8, wherein the phthalocyanine crystal is a hydroxygallium phthalocyanine crystal.

10. The photoelectric conversion element according to any one of claims 1 to 9, wherein the phthalocyanine crystal contains N-methylformamide.

11. The photoelectric conversion element according to any one of claims 1 to 10, wherein an average thickness of the charge-transporting layer is 50 to 180 nm.

12. A photoelectric conversion apparatus comprising the photoelectric conversion element of any one of claims 1 to 11.

13. A moving body comprising the photoelectric conversion element of any one of claims 1 to 11.

14. A building material comprising the photoelectric conversion element of any one of claims 1 to 11.
